# EUROPEAN PATENT SPECIFICATION

(11) **EP 1 674 054 B1**
(45) Date of publication and mention of the grant of the patent: **14.05.2008**
(21) Application number: 05107325.2
(22) Date of filing: 09.08.2005
(51) Int. Cl.: A61F 5/00

(54) **Functional glove for the hand of a disabled person**
Funktionshandschuh für menschliche Hand einer behinderten Person
Gant fonctionnel pour les mains d'une personne handicapée

(30) Priority: 13.08.2004 IT BO20040528
(43) Date of publication of application: 28.06.2006
(73) Proprietor: Meola, Elena, 40136 Bologna (IT); Meola, Cristina, 40033 Casalecchio di Reno (BO) (IT)
(72) Inventor: Vannini, Antonietta, deceased (IT)
(74) Representative: Modiano, Micaela Nadia

(56) References cited:
- FR-A- 2 822 371
- US-A- 4 765 320
- US-A- 5 466 215
- US-A- 5 771 901
- US-A- 5 807 293
- US-B1- 6 475 174

## Description

The present invention relates to a functional glove for the hand of a disabled person.

Quadriplegia compromises manual functionality by reducing the number of motor operations, which are usually required in common activities of everyday life for self-management and for interaction with the microenvironment that surrounds the patient.

Manual disability entails assistance of the patient even for meeting the most basic needs, from personal hygiene to feeding to graphical communication to pushing the wheelchair in all the various transfers.

The severity of manual disability has always promoted research aimed at giving back at least partially to the patient this basic gestural expressiveness.

Highly diversified solutions have thus been obtained which range from the construction of small aids which facilitate manual motor operations to important technologies which propose to animate the hand by means of functional electrical stimulation systems.

The described aids substantially comprise all the commonly used objects to which a particular diversified shape has been given (in order to facilitate their grip even if the patient is unable to close his fist) or which are provided with receptacles in which one or more fingers or the entire hand are accommodated. In practice, the hand and the tool are coupled artificially (i.e., without using the normal grip of the hand).

The hypothetical case of functional electrical stimulation instead entails implanting a plurality of electrodes at the muscle surface in the motor points; said electrodes are driven by a stimulator (which also can be optionally implanted). By way of the presence of a sensor for detecting motion, generally of the shoulder of the injured limb, the simulator provides an electrical signal to the electrodes: for example, a retraction of the shoulder can be interpreted by the stimulator as the command to close the fist.

None of these solutions is easily accepted and used by the patient for various reasons, such as the slowness of the motor operation induced by the aid or the management complexity of the tool (especially in the case of neuroprostheses with electrical stimulation).

The patient in fact needs the manual gesture to be performed even inaccurately but in time with his motor requirements. For this reason, many solutions which are scientifically interesting are not requested and used by the patient in practice.

US Patent No. 5,807,293 discloses a splint assembly for a human hand splinting and/or orthopedic assembly device used to position a disabled, diseased or injured human hand. The splint assembly is provided for supporting the user's wrist in a neutral or relatively extended configuration with the user's phalanges in a normal functional position.

The aim of the present invention is to obviate the cited drawbacks and meet the mentioned requirements, by providing a functional glove for the hand of a disabled person by way of which it is possible to prearrange the hand of the patient in order to facilitate the handling of various objects.

An object of the present invention is to mass-produce the glove according to the invention, since it can be adapted to any possible disability of the patient.

Another object of the present invention is to provide a glove whose shape and dimensions are similar to those of gloves used for normal sports applications, and which therefore does not have the appearance of an auxiliary apparatus for handicapped individuals and is therefore similar to the gloves normally used by the disabled to make it easier for them to push the wheelchair.

Within this aim, another object of the present invention is to provide a structure which is simple, relatively easy to provide in practice, safe in use, effective in operation, and has a relatively low cost.

This aim and this object are achieved by the present functional glove for the hand of a disabled person,

Further characteristics and advantages will become better apparent from the following detailed description of a preferred but not exclusive embodiment of a functional glove for the hand of a disabled person, illustrated by way of non-limiting example in the accompanying drawings, wherein:

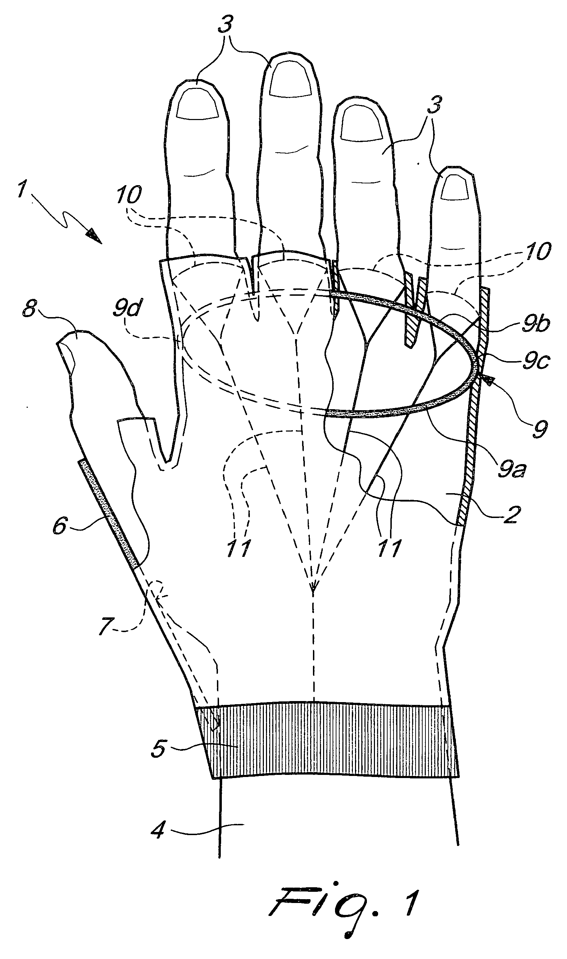

Figure 1 is a plan view of a glove according to the invention, worn on a hand;

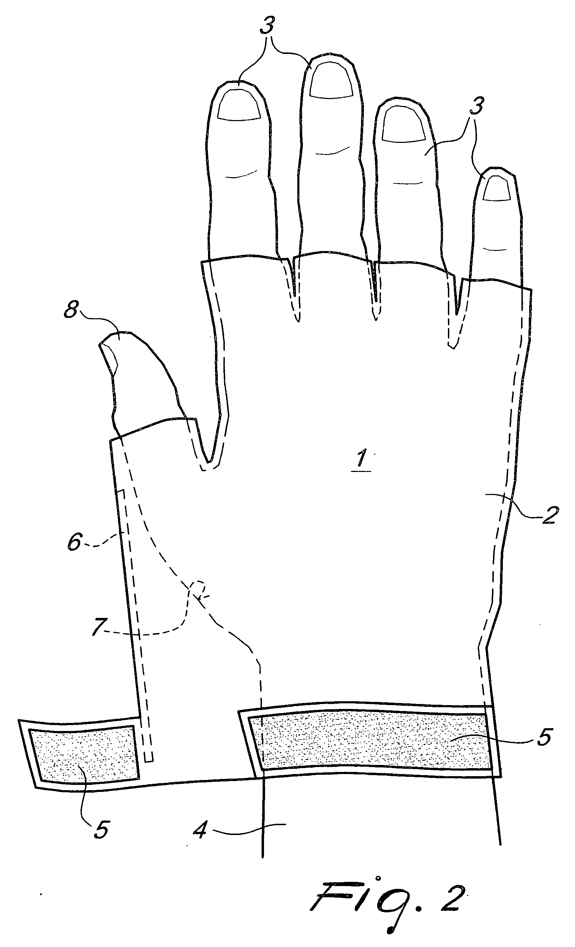

Figure 2 is a plan view of a glove according to the invention in the open configuration, with the wrist free.

With reference to the figures, the reference numeral 1 generally designates a functional glove for the hand of a disabled person.

The functional glove 1 is suitable to be fitted over the hand 2, leaving free the tips of the fingers 3, and is then fixed firmly to the wrist 4 by means of a fastening strip 5 made of a hook and loop-fastener known as Velcro^{®}.

The glove comprises a substantially elastic splint 6, which is arranged on the lateral surface of the glove 1, starting from the wrist 4, along with the lateral surface of the short adductor 7 of the thumb 8, up to the upper portion of the thumb 8. The splint 6 can be fixed directly by sewing it on to the glove 1 (or can be rigidly coupled with other similar solutions), but it is also possible to provide a hollow seat (formed by stitching a flap of fabric onto the glove 1, forming a pocket), in which the splint 6 is inserted. The splint 6 is locked to the wrist because its end that is aligned with said wrist is accommodated below the strip of Velcro 5.

The presence of the splint 6 ensures that the thumb 8 of the hand 2 of the disabled person maintains an active position, because the splint 6 is substantially uncurved, i.e. straight and therefore forces alignment between the wrist 4 and the portion of the thumb 8 to which it is fixed: positioning the splint 6 on one side or the other of the wrist 4 allows to arrange the thumb 8 in opposition to the other fingers 3 (in order to allow easier grip) or align it with them (for example to push the wheelchair easily).

An ellipsoidal body 9 is fitted on the back of the glove 1, on the proximal and distal part of the metacarpal phalangeal articulations of the first four fingers 3, and is anchored by means of an annular structure (not shown in the figure) to the palm portion of the glove 1.

The portion 9a of the ring 9, when the glove is fitted, is located on the proximal part of the metacarpal phalangeal articulation, while portion 9b lies above the distal part: the articulation is comprised within the ring 9.

The ends 9c and 9d of the ring 9 are fixed adjustably to the annular structure that is provided on the palm portion: by adjusting the length of this fixing, it is possible to force the ring 9 more or less strongly onto the hand 2, changing the opening angle of the metacarpal phalangeal articulation and preparing the hand 2 of the disabled patient in a position that facilitates grip.

Each receptacle for the fingers 3 has, at its end, at least one sort of small noose 10, which is rigidly coupled to a respective bridle 11, which can slide on the back of the glove 1 and has an adjustable length.

In practice, each bridle 11 surrounds, with the respective end noose 10, a receptacle for a finger 3 of the glove 1 at the phalangeal articulation, and its opposite end is rigidly coupled to the Velcro strip 5 and is therefore rigidly coupled to the wrist 4 by fastening it.

The optimum execution of a glove 1 according to the invention provides for a pair of nooses 10 for each finger 3, each noose being connected to a respective bridle 11: the bridle 11 that leads to a first noose 10 preferably slides within the glove 1 over the back of the hand 2, and the other bridle 11, which leads to the second noose 10 (arranged opposite the first one), slides inside the glove 1 below the palm of the hand 2. By pulling the finger 3 with one bridle 11 or the other, it is possible to determine the most suitable position thereof.

The elastic splint 6, the ellipsoidal body 9 and the bridles 11 are provided simultaneously on the glove 1: their use or action can be simultaneous or separate (independently of each other) depending on the requirements of the patient.

In order to facilitate the insertion of the hand 2, the glove 1 is made of a material whose internal surface has high surface sliding properties.

The operation of the invention is intuitive: once the glove 1 has been put on, the thumb 8 of the patient is kept in a partially open position or in opposition to the other fingers 3 (depending on the position for fixing to the wrist 4 and on the elastic action performed by the splint 6) as a consequence of the presence of the splint 6. The four fingers 3, which a disabled person normally tends to keep with their metacarpal phalangeal articulation hyperextended, are kept in a partially bent position with respect to the palm of the hand 2 as a consequence of the presence of the ring 9: the angle at which the metacarpal phalangeal articulation is arranged depends on the extent to which the ends 9c and 9d are fastened to the annular structure that lies on the palm portion of the glove 1.

Each finger 3, considered individually, is kept in a preset position by the respective bridles 11, being surrounded by the nooses 10 and kept under traction as a consequence of the fixing of the opposite end of the bridles 11 to the Velcro band 5. This prevents the patient, as a consequence of involuntary movements, from closing a finger 3 onto the palm of the hand 2, hindering grip.

It should be noted that disabled patients normally wear gloves such as the ones used by cyclists in order to improve friction between the hand 2 and the respective wheel of the wheelchair: wearing a functional glove 1 according to the invention therefore does not entail any psychological burden for the patient, since the glove 1 is shaped in a manner which is substantially similar to a cycling glove.

Optional alternative embodiments may allow at least one of the aids (splint 6, ring 9 and bridles 11) to be removable if the patient does not require their action, and it is likewise possible to provide a glove 1 that has only one or two of these aids.

It has thus been shown that the invention achieves the proposed aim and objects.

The invention thus conceived is susceptible of numerous modifications and variations, all of which are within the scope of the appended claims.

All the details may further be replaced with other technically equivalent ones.

In the embodiments shown, individual characteristics, given in relation to specific examples, may actually be interchanged with other different characteristics that exist in other embodiments.

Moreover, it is noted that anything found to be already known during the patenting process is understood not to be claimed and to be the subject of a disclaimer.

The embodiment of the present invention shall be carried out in the most scrupulous compliance with the statutory and regulatory provisions related to the products of the invention or correlated thereto and following any required authorization of the corresponding competent authorities, with particular reference to regulations related to safety, environmental pollution and health.

In practice, the materials used, as well as the shapes and dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A functional glove for the hand (2) of a disabled person, comprising at least one flexible restraint for at least one of the fingers (3) of the hand (2), and removable fastening means (5) for rigid fixing to the wrist (4), said glove (1) being worn over the hand (2) by the disabled person during use, **characterized in that**
said restraint is an ellipsoidal body (9), which is designed to be arranged on the proximal and distal part of the metacarpal phalangeal articulations of the first four fingers (3) and is anchored, by means of an annular structure, to the palm portion of said glove (1).

2. The glove according to claim 1, **characterized in that** said restraint is an elastic splint (6), which is arranged on the lateral surface of the glove (1), designed to start from the wrist (4), then to extend along the lateral surface of the short adductor (7) of the thumb (8), up to the upper portion of said thumb (8), and is rigidly coupled to said glove (1), being lockable to the wrist (4) by way of said fastening means (5).

3. The glove according to claim 2, **characterized in that** said splint (6) is linear, not curved, and suitable to keep the thumb (8) in different positions which depend on the position in which said splint is fixed to the wrist (4).

4. The glove according to claim 1, **characterized in that** said restraint is constituted by at least one bridle (11) of adjustable length, which surrounds a respective receptacle for a finger of said glove (1) at a phalangeal articulation with an end thereof which is shaped like a noose (10), the opposite end being rigidly coupled to said fastening means (5).

5. The glove according to claim 4, **characterized in that** said bridles (11) are four, each one being arranged at a respective receptacle for a finger (3) of said glove (1).

6. The glove according to claim 4, **characterized in that** said bridles (11) are eight, each pair of said bridles being connected to a respective noose (10), which is arranged at a respective receptacle for a finger (3) of said glove (1), a first bridle (11) being able to slide over the back of the hand (2) and a second bridle (11) being able to slide under the palm of the hand (2).

7. The glove according to claims 1, 2 and 4, **characterized in that** said elastic splint (6), said ellipsoidal body (9) and said bridles (11) are simultaneously present on said glove (1), said restraints being designed to be functionally associated with the hand (2) that wears the glove according to the requirements, even independently of each other.

8. The glove according to claim 1, **characterized in that** said removable fastening means (5) for rigid fixing to the wrist (4) comprise a strip of Velcro^{®}.

9. The glove according to one or more of the preceding claims, further comprising receptacles suitable to accommodate the fingers (3), **characterized in that** said receptacles do not have a tip, at least the last phalanx of each finger (3) being free when the glove (1) is being worn.

10. The glove according to one or more of the preceding claims, **characterized in that** it is made of a material having a highly sliding internal surface in order to facilitate insertion of the hand (2).

## Patentansprüche

1. Ein funktioneller Handschuh für die Hand (2) einer behinderten Person, der mindestens eine flexible Halterung für mindestens einen der Finger (3) der Hand (2) und lösbare Befestigungsmittel (5) zur starren Befestigung am Handgelenk (4) umfasst, wobei der Handschuh (1) während des Gebrauchs durch die behinderte Person über der Hand (2) getragen wird, **dadurch gekennzeichnet, dass**
die Halterung ein ellipsoider Körper (9) ist, der konstruiert ist, um auf dem proximalen und distalen Teil der metakarpophalangealen Gelenke der ersten vier Finger (3) angeordnet zu werden und der mittels einer ringförmigen Struktur am Handflächen-Abschnitt des Handschuhs (1) verankert wird.

2. Der Handschuh gemäß Anspruch 1, **dadurch gekennzeichnet, dass** die Halterung eine elastische Schiene (6) ist, welche an der seitlichen Oberfläche des Handschuhs (1) angebracht ist, konstruiert, um vom Handgelenk (4) auszugehen, sich dann entlang der seitlichen Oberfläche des kurzen Adduktors (7) des Daumens (8), bis zum oberen Abschnitt des Daumens (8), zu erstrecken, und welche mit dem Handschuh (1) starr verbunden ist, wobei sie am Handgelenk (4) mit Hilfe des Befestigungsmittels (5) befestigt werden kann.

3. Der Handschuh gemäß Anspruch 2, **dadurch gekennzeichnet, dass** die Schiene (6) linear, nicht gekrümmt, und geeignet ist, den Daumen (8) in verschiedenen Positionen zu halten, die von der Position abhängen, in welcher die Schiene am Handgelenk (4) befestigt wird.

4. Der Handschuh gemäß Anspruch 1, **dadurch gekennzeichnet, dass** die Halterung aus mindestens einer Bride (11) einstellbarer Länge besteht, die eine entsprechende Aufnahme für einen Finger des Handschuhs (1) an einem phalangealen Gelenk mit einem Ende davon umgibt, die wie eine Schlinge (10) geformt ist, wobei das gegenüberliegende Ende starr mit dem Befestigungsmittel (5) verbunden ist.

5. Der Handschuh gemäß Anspruch 4, **dadurch gekennzeichnet, dass** es vier Briden (11) gibt, wobei jede an einer entsprechenden Aufnahme für einen Finger (3) des Handschuhs (1) angeodnet ist.

6. Der Handschuh gemäß Anspruch 4, **dadurch gekennzeichnet, dass** es acht Briden (11) gibt, wobei jedes Paar der Briden mit einer entsprechenden Schlinge (10) verbunden ist, die an einer entsprechenden Aufnahme für einen Finger (3) des Handschuhs (1) angebracht ist, wobei eine erste Bride (11) über den Rücken der Hand (2) gleiten kann und eine zweite Bride (11) unter der Handfläche der Hand (2) gleiten kann.

7. Der Handschuh gemäß Anspruch 1, 2 und 4, **dadurch gekennzeichnet, dass** die elastische Schiene (6), der ellipsoide Körper (9) und die Briden (11) gleichzeitig an dem Handschuh (1) vorhanden sind, wobei die Halterungen konstruiert sind, um entsprechend den Erfordernissen funktionell mit der Hand (2) verbunden zu werden, die den Handschuh trägt, selbst unabhängig voneinander.

8. Der Handschuh gemäß Anspruch 1, **dadurch gekennzeichnet, dass** die lösbaren Befestigungsmittel (5) zur starren Befestigung am Handgelenk (4) einen Streifen Velcro^{®} umfassen.

9. Der Handschuh gemäß einem oder mehreren der obigen Ansprüche, der weiter Aufnahmen umfasst, die geeignet sind, die Finger (3) aufzunehmen, **dadurch gekennzeichnet, dass** die Aufnahmen keine Spitze haben, wodurch mindestens das letzte Glied jedes Fingers (3) freiliegt, wenn der Handschuh (1) getragen wird.

10. Der Handschuh gemäß einem oder mehreren der obigen Ansprüche, **dadurch gekennzeichnet, dass** er aus einem Material besteht, das eine stark gleitende innere Oberfläche hat, um das Einführen der Hand (2) zu erleichtern.

## Revendications

1. Gant fonctionnel pour la main (2) d'une personne handicapée, comprenant au moins un organe de contrainte flexible pour au moins l'un des doigts (3) de la main (2), et des moyens de fixation amovibles (5) pour une fixation rigide au poignet (4), ledit gant (1) étant enfilé sur la main (2) par la personne handicapée durant l'utilisation, **caractérisé en ce que** ledit organe de contrainte est un corps ellipsoïdal (9) qui est étudié pour être agencé sur la partie distale et proche des articulations métacarpo-phalangiennes des quatre premiers doigts (3) et est fixé, au moyen d'une structure annulaire, à la partie palmaire dudit gant (1).

2. Gant selon la revendication 1, **caractérisé en ce que** ladite contrainte est une attelle élastique (6) qui est agencée sur la surface latérale du gant (1), étudiée pour partir du poignet (4) et pour s'étendre ensuite le long de la surface latérale de l'abducteur court (7) du pouce (8), au dessus de la partie supérieure du pouce (8) et est rigidement couplée audit gant (1), pouvant être bloquée au poignet (4) au moyen desdits moyens de fixation (5).

3. Gant selon la revendication 2, **caractérisé en ce que** ladite attelle (6) est linéaire, non courbe, et adaptée pour maintenir le pouce (8) dans différentes positions qui dépendent de la position dans laquelle ladite attelle est fixée audit poignet (4).

4. Gant selon la revendication 1, **caractérisé en ce que** ladite contrainte est constitué par au moins une bride (11) à longueur réglable, qui ceint un réceptacle respectif pour un doigt dudit gant (1) au niveau d'une articulation phalangienne avec une extrémité de celui-ci qui a la forme d'un noeud coulant (10), l'extrémité opposée étant rigidement couplée auxdits moyens de fixation (5).

5. Gant selon la revendication 4, **caractérisé en ce que** lesdites brides (11) sont quatre, chacune étant agencée au niveau d'un réceptacle respectif pour un doigt (3) dudit gant (1).

6. Gant selon la revendication 4, **caractérisé en ce que** lesdites brides (11) sont huit, chaque paire desdites brides étant reliée à un noeud coulant respectif (10), qui est agencée au niveau d'un réceptacle respectif pour un doigt (3) dudit gant (1), une première bride (11) étant apte à coulisser sur le dos de la main (2) et une deuxième bride (11) étant apte à coulisser sous la paume de la main (2).

7. Gant selon les revendications 1, 2 et 4, **caractérisé en ce que** ladite attelle élastique (6), ledit corps ellipsoïdal (9) et lesdites brides (11) sont simultanément présentes sur ledit gant (1), lesdites contraintes étant étudiées pour être associées de manière fonctionnelle à ladite main (2) sur laquelle est enfilé le gant selon les nécessités, même indépendamment l'une de l'autre.

8. Gant selon la revendication 1, **caractérisé en ce que** lesdits moyens de fixation amovibles (5) pour la fixation rigide au poignet (4) comprennent une bande Velcro®.

9. Gant selon une ou plusieurs des revendications précédentes, comprenant en outre des réceptacles appropriés pour loger les doigts (3), **caractérisé en ce que** lesdits réceptacles ne présentent pas de pointe, au moins la dernière phalange de chaque doigt (3) étant libre lorsque le gant (1) est enfilé.

10. Gant selon une ou plusieurs des revendications précédentes, **caractérisé en ce qu'**il est fait d'un matériau présentant une surface interne hautement coulissante afin de faciliter l'insertion de la main (2).
